# EUROPÄISCHE PATENTANMELDUNG

(11) **EP 2 937 103 A1**
(43) Veröffentlichungstag der Anmeldung: **28.10.2015**
(21) Anmeldenummer: 15164725.2
(22) Anmeldetag: 22.04.2015
(51) Int. Cl.: A61L 2/14, C23C 18/14, H05H 1/24

(54) **WIRKAUFLAGE UND VERFAHREN ZUR BEHANDLUNG EINER OBERFLÄCHE**

(30) Priorität: 23.04.2014 DE 102014105720
(71) Anmelder: INP Greifswald E.V., 17489 Greifswald (DE)
(72) Erfinder: Weltmann, Klaus-Dieter, 18609 Binz (DE); Dr. René, Bussiahn, 14793 Greifswald (DE); Winter, Dr. Jörn, 17489 Greifswald (DE); von Woedtke, Thomas, 18519 Gerdeswalde (DE)
(74) Vertreter: Eisenführ Speiser

(57) **Zusammenfassung**

Wirkauflage (2) mit einem Wirkmedium (4), dadurch gekennzeichnet, dass das Wirkmedium (4) zur Aktivierung durch Plasma und zur Freisetzung eines Wirkstoffs nach der Plasmaaktivierung ausgebildet ist.

## Beschreibung

Die Erfindung betrifft eine Wirkauflage mit einem Wirkmedium.

Die Erfindung betrifft weiterhin ein Verfahren zur Behandlung einer Oberfläche mit einer solchen Wirkauflage.

Es ist insbesondere aus WO 2009/101143 A1 bekannt, eine antimikrobielle Behandlung von Oberflächen und Kavitäten zur Entkeimung, Desinfektion, Sterilisation oder Dekontamination (zusammengefasst nachfolgend als Desinfektion bezeichnet) mittels einem kalten Atmosphärendruck-Plasma durchzuführen. Hierbei wird ein Plasmagasstrom direkt auf die Oberfläche geleitet.

Beispielsweise aus WO 2011/063798 A2 ist eine Vorrichtung in einem Verfahren zur Erzeugung eines gepulsten anisothermen Atmosphärendruck-Plasmas zu einer punktgenauen mikrobiellen Plasmabehandlung kleinster Flächen und Kavitäten bekannt.

Auch in der WO 2012/163876 A1 ist eine Vorrichtung zum Erzeugen eines stark fokussierten Plasmastroms beschrieben.

Die Wechselwirkung von Plasma mit Flüssigkeiten und die daraus resultierende bakterizide Wirkung ist Gegenstand diverser wissenschaftlicher Veröffentlichungen. So zeigen K. Oehmigen et al.: The Role of Acidification for Antimicrobial Activity of Atmospheric Pressure Plasma in Liquids, In: Plasma Proc. Polym., 2010, dass durch Plasmabehandlung mittels einer dielektrisch behinderten Entladung langlebige aktive Spezies wie beispielsweise H₂O₂ oder Nitrit oder Nitrat innerhalb der Flüssigkeit produziert werden. Dieser plasmaaktivierten Flüssigkeit wurden nach Abschluss der Plasmabehandlung Bakterien ausgesetzt und ein bakterizider Effekt beobachtet.

J. Winter et al.: Feed Gas Humidity: A Vital Parameter Affecting a Cold Atmospheric-pressure Plasma Jet and Plasma-treated Human Skin Cells, In: Phys. D: Appl. Phys., 2013 zeigten in einem ähnlichen Versuch die Inhibierung von humanen Hautzellen durch indirekte Plasmabehandlung, indem Zellkulturmedium mit einem Atmosphärendruck-Argon-Plasmajet behandelt und anschließend die behandelte Flüssigkeit auf humane Hautzellen gegeben wurden. Hier wurde Wasserstoffperoxid als wesentliche biologisch aktive Komponente identifiziert.

Zum gleichen Schluss kamen S. Bekeschuss et al.: Hydrogen Peroxide: A Central Player, In: Physical Plasma-induced Oxidative Stress in Human Blood Cells, In: Free radical research, 2014, die humane Immunzellen in einer Zellkulturlösung mit dem gleichen Plasmajet behandelten.

Ein Beispiel für eine direkte Plasmabehandlung zum Zweck der Bakterieninaktivierung innerhalb einer Flüssigkeit liefern Fuxian Liu et al.: Inactivation of Bacteria, In: An Aqueous Environment by a Direct-Current, Cold-Atmospheric-Pressure Air Plasma Microjet, In: Plasma Proc. Polym., 2010. Im Gegensatz zu den vorher beschriebenen Arbeiten wurde das Plasma innerhalb der Flüssigkeit gezündet und eine bakterizide Wirkung festgestellt.

Ein Literaturüberblick über die derzeit bekannten Wirkmechanismen von plasmagenerierten Spezies (reactive oxygen and nitrogen spezies) in bezug auf biomedizinische Anwendungen ist in D. Graves: The Emerging Role of Reactive Oxygen and Nitrogen Species in Redox Biology and Some Implications for Plasma Applications to Medicine an Biology, In: J. Phys. D.: Appl. Phys., 2012, 45, gegeben.

Weiterhin ist es zur Wundversorgung bekannt, mit Wirkstoff getränkte oder beschichtete Wundauflagen zu verwenden oder die mit Salben versorgte Wunde mit einem Verband abzudecken (siehe C. Erfurt-Berge und R. Renner: Recent Developments in Topical Wound Therapy: Impact of Antimicrobiological Changes and Re-balancing the Wound Milieu, In: BioMed Research International, 2014).

Aus Yasushi Sasai, Shin-ichi Kondo, Yukinori Yamauchi, Masayuki Kuzuya: Cold Plasma Techniques for Pharmaceutical and Biomedical Engineering, In: Trends in Materials Science, Intech, 2011 ist die Plasmabehandlung eines Polymermantels für ein Medikament zur verbesserten Wirkstoffabgabe bei Tabletteneinnahme beschrieben.

Ausgehend hiervon ist es Aufgabe der vorliegenden Erfindung eine verbesserte Wirkauflage und ein verbessertes Verfahren zur Behandlung einer Oberfläche zu schaffen, um die Einwirkung auf eine größere Fläche über eine hinreichende Zeit zu ermöglichen und dabei auf einfache und sichere Weise einen Wirkstoff zu erzeugen und/oder die Wirkstofffreigabe zu verbessern.

Die Aufgabe wird mit der Wirkauflage mit den Merkmalen des Anspruchs 1 und durch das Verfahren mit den Merkmalen des Anspruchs 9 gelöst. Vorteilhafte Ausführungsformen sind in den Unteransprüchen beschrieben.

Es wird vorgeschlagen, dass das Wirkmedium der Wirkauflage zur Aktivierung durch Plasma und zur Freisetzung eines Wirkstoffs nach der Plasmaaktivierung ausgebildet ist.

Damit kann z.B. eine durch die bisherige direkte Plasmabehandlung einer Oberfläche bewirkte desinfizierende Wirkung indirekt durch Übertragung der Plasmaeigenschaft auf das Wirkmedium erzielt werden. Die Oberfläche muss damit nicht direkt dem Plasmastrom ausgesetzt werden, was die Handhabung und die Sicherheit verbessert. Durch die Aktivierung eines Wirkmediums mittels Plasma gelingt es, relativ große Flächen für eine längere Zeit zu behandeln und diesen Flächen indirekt die Wirkung des Plasmas zukommen zu lassen.

Ein kaltes Atmosphärendruck-Plasma wird in der Regel mit einer Hochspannungsquelle in einem Gas erzeugt, so dass die zu behandelnde Oberfläche in die Nähe der Hochspannungsquelle kommt oder dem energiehaltigen Gas direkt ausgesetzt wird.

Durch die Aktivierung des Wirkmediums mittels des Plasmas wird nunmehr lediglich das Wirkmedium dem Plasma ausgesetzt. Es wird anschließend auf die Oberfläche appliziert, sodass die Anwendung vereinfacht wird und wesentlich sicherer ist.

Dadurch, dass die Wirkstoffe nunmehr vom Plasma direkt in einem Medium erzeugt, oder in ein Medium transferiert, dort kurzzeitig gespeichert und kontrolliert freigesetzt werden, wird eine aktive positive Beeinflussung der Oberfläche z. B. zur Wundheilung ermöglicht. Zudem wird eine längere Einwirkzeit bei kontrollierter Wirkstofffreisetzung ermöglicht.

Die Wundauflage kann hierzu ein plasmaaktivierbares Gel, eine plasmaaktivierbare Flüssigkeit oder eine plasmaaktivierbare fluidgetränkte Trägerschicht haben. Durch die Plasmabehandlung des Wirkmediums in Gelform, flüssiger Form oder als fluidgetränkte Trägerschicht wird darin ein Wirkstoff erzeugt oder freigesetzt.

Als Wirkmedium sind wasserbasierte Flüssigkeiten, Kochsalzlösungen und davon abgeleitete Gele geeignet.

Alternativ oder zusätzlich zu der Bereitstellung eines Wirkmediums in fester oder flüssiger Form ist denkbar, dass die Wirkauflage einen mit Plasma generierter Spezies (z.B. gas- oder fluidförmig) befüllbaren Entladungsraum hat. Ein im oder angrenzend an einen Entladungsraum befindliches Wirkmedium wird dann durch Zuführung der plasmagenerierten Spezies in den Entladungsraum aktiviert, um sodann an die zu behandelnde Oberfläche freigesetzt zu werden.

Die Wirkauflage kann beispielsweise eine mit dem Wirkmedium gekoppelte Elektrodenanordnung zur Plasmaaktivierung des Wirkmediums haben. Dabei sind elektrische Kontakte zum Anschluss der Elektrodenanordnung an einen von der Wirkauflage separaten Hochspannungsgenerator vorgesehen. Zur Aktivierung des Wirkmediums wird der Hochspannungsgenerator an die noch nicht auf die Oberfläche applizierte Wundauflage angeschlossen. Dann wird die Elektrode mit einer Hochspannung in Form einer Gleichspannung, einer gepulsten Spannung oder einer Wechselspannung beaufschlagt, um ein Plasma, bevorzugt ein kaltes Atmosphärengasplasma, zu erzeugen und dessen Agentien auf das Wirkmedium zu übertragen. Nach Aktivierung des Wirkmediums wird die elektrische Verbindung zwischen dem Hochspannungsgenerator und der Wirkauflage getrennt und die Wirkauflage auf die zu behandelnde Oberfläche appliziert.

In entsprechender Weise ist auch denkbar, dass die Wirkauflage einen mit dem Wirkmedium kommunizierend verbundenen Gasanschluss zum Anschluss einer von der Wirkauflage separaten Plasmaversorgungseinheit für die Zuleitung von plasmagenerierter aktiver Spezies (z.B. als Gas oder Fluid) von der Plasmaversorgungseinheit zum Wirkmedium hat.

Bei der erfindungsgemäßen Wirkauflage wird somit die Plasmabehandlung zur Aktivierung des Wirkmediums von der Behandlung der Oberfläche zeitlich und räumlich getrennt. Die zu behandelnde Oberfläche hat somit zu keinem Zeitpunkt einen Kontakt zu dem zur Aktivierung des Wirkmediums genutzten Plasma. Dabei kann das plasmaaktivierte Wirkmedium ohne den direkten Einsatz des Plasmas an der Oberfläche angewendet werden. Die Oberfläche wird damit keinen elektrischen Gefahren und keiner Strahlung ausgesetzt. Die Wirkstofffreigabe wird dabei durch das Wirkmedium und die Ausgestaltung der Desinfektionsauflage gesteuert.

Denkbar ist auch, dass die Wundauflage ein das Wirkmedium tragendes Verbandsmaterial hat und zur Auflage auf die zu behandelnden Oberfläche vorgesehen ist. Das Wirkmedium kann dann als Desinfektionsauflage auf einer Oberfläche aufgebracht werden, um diese Oberfläche zu desinfizieren und abzudecken. Es kann auch als Wundverband auf eine Wundoberfläche aufgebracht und dort fixiert werden, um so die Wundheilung zu fördern.

Das Wirkmedium ist bevorzugt mikrobiologisch desinfizierend.

Zur Behandlung der Oberfläche wird das Wirkmedium der Wirkauflage durch Beaufschlagung des Wirkmediums mit einem Plasma aktiviert und zeitlich und räumlich getrennt davon anschließend das Wirkmedium auf die zu behandelnde Oberfläche durch Auflegen der Wirkauflage auf die zu behandelnde Oberfläche appliziert.

Die Erfindung wird nachfolgend anhand der beigefügten Zeichnungen näher erläutert. Es zeigen:
- Fig. 1: - Skizze der Behandlung einer Oberfläche mit einer Wirkauflage, dessen Wirkmedium von außen mit einer separaten Plasmaquelle aktiviert wird;
- Fig. 2: - Skizze der Behandlung einer Oberfläche mit einer Wirkauflage, die einen Entladungsraum zur Erzeugung plasmagenerierter aktiver Spezies hat;
- Fig. 3: - Skizze der Behandlung einer Oberfläche mit einer Wirkauflage, die zur Plasmaaktivierung eine Elektrodenanordung mit einem Anschluss eines Hochspannungsgenerators hat;
- Fig. 4: - Skizze der Behandlung einer Oberfläche mit einer Wirkauflage, die einen Anschluss für eine Plasmaversorgungseinheit zur Zufuhr von plasmagenerierter aktiver Spezies hat;
- Fig. 5: - Schnittansicht einer Wirkauflage mit einem Träger, einem Auflagekissen, einer Membran und einem dazwischen angeordneten plasmaaktivierbarem Wirkmedium;
- Fig. 6: - Schnittansicht einer Wirkauflage mit einem Träger und einer mit einer mit plasmaaktivierbarem Wirkmedium getränkten Wirkschicht.

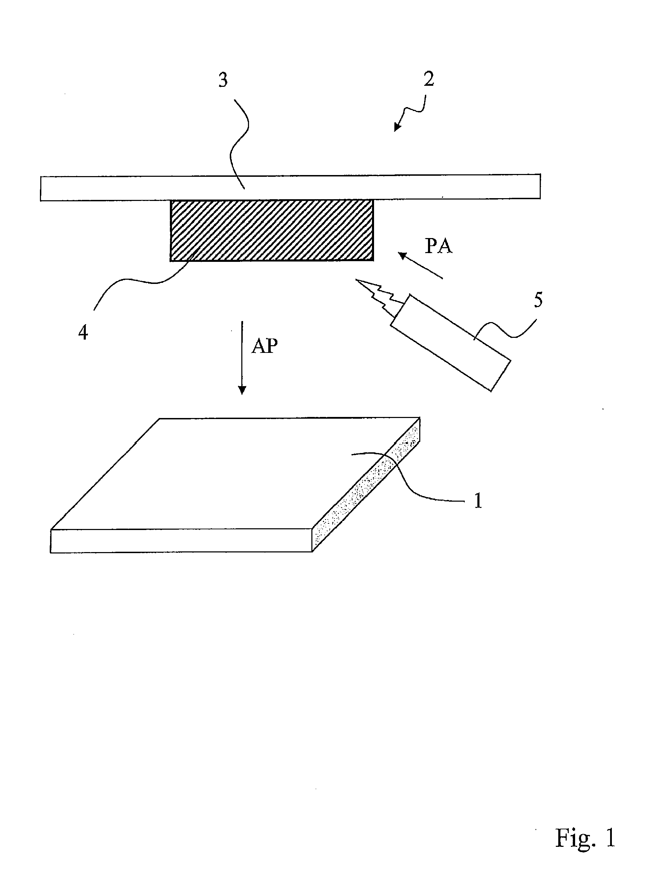

Figur 1 lässt eine Skizze der Behandlung einer Oberfläche 1 mit einer Wirkauflage 2 erkennen, die einen flächigen Träger 3 hat, der ein Wirkmedium 4 trägt. Das Wirkmedium 4 hat je nach ausgewählter Zusammensetzung eine spezifische Wirkung, die erst nach Aktivierung mit einem Plasma eintritt. So kann das Wirkmedium 4 z.B. eine mikrobiologisch desinfizierende Wirkung haben, nachdem das Wirkmedium 4 einem Plasma ausgesetzt wurde.

In dem dargestellten Ausführungsbeispiel ist skizziert, dass in einem Aktivierungsschritt PA (PlasmaAktivierung) das Wirkmedium 4 mittels einer Plasmagenerators 5, der z.B. ein kaltes Atmosphärendruckplasma erzeugt, aktiviert wird. Hierzu wird das Plasma bevorzugt flächig auf das Wirkmedium 4 appliziert. Dieses aktivierte Wirkmedium 4 wird sodann in einem Applikationsschritt AP auf die zu behandelnde Oberfläche 1 aufgetragen. Damit können die Wirkeigenschaften von Plasma im Vergleich zur der direkten Plasmabehandlung einer Oberfläche 1 nunmehr indirekt über das Wirkmedium auf größere Oberflächen 1 und für einen längeren Zeitraum appliziert werden.

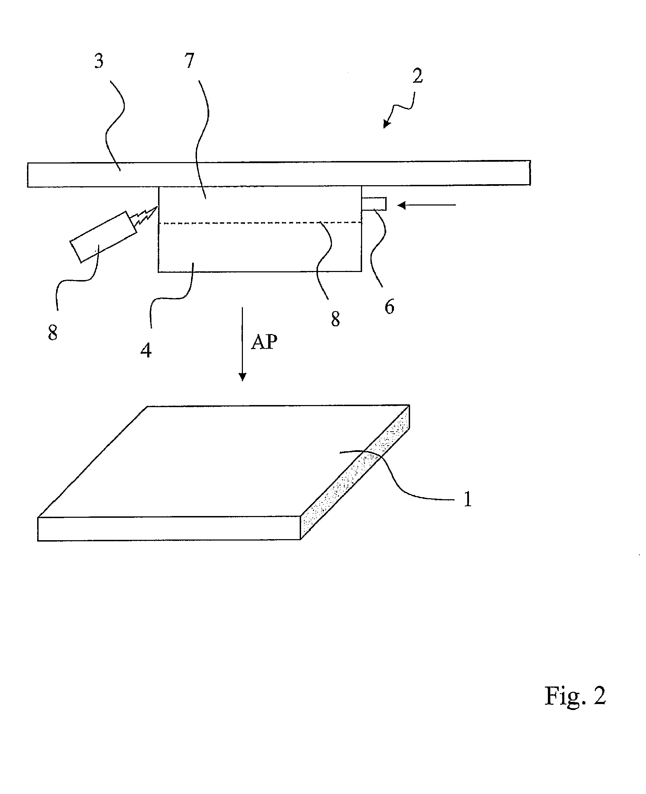

Figur 2 lässt eine Skizze der Behandlung einer Oberfläche 1 mit einer Wirkauflage 2 erkennen, die einen über einen Gasanschluss 6 mit Gas befüllbaren Entladungsraum 7 hat. Dieses Gas kann dann z.B. durch Anschluss einer Zündquelle 8 an den Entladungsraum 7 gezündet werden, um ein Plasma zu erzeugen. Angrenzend an den Entladungsraum 7 befindet sich das Wirkmedium 4, das beispielsweise über eine Membran 8 getrennt sein kann und derart in Verbindung mit dem Entladungsraum 7 steht, dass das Wirkmedium 4 nach Zünden des Gases im Entladungsraum 7 durch das so erzeugte Plasma aktiviert wird.

Wiederum wird dieses aktivierte Wirkmedium 4 sodann in einem Applikationsschritt AP durch Auflegen der Wirkauflage 2 auf die zu behandelnde Oberfläche 1 auf die Oberfläche 1 appliziert.

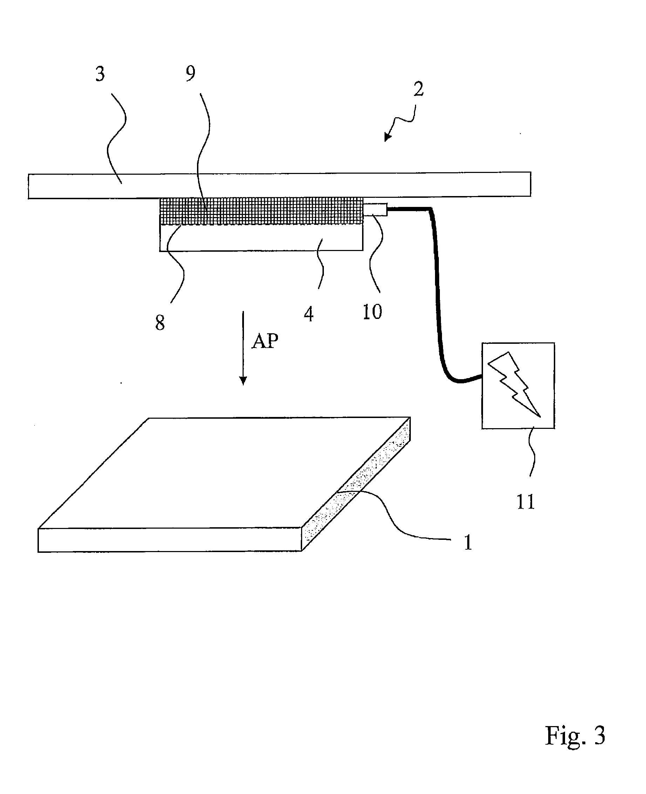

Figur 3 zeigt eine Skizze einer weiteren Ausführungsform zur Behandlung einer Oberfläche 1 mit einer Wirkauflage 2, die nunmehr zur Plasmaaktivierung eine Elektrodenanordung 9 an einem Träger 3 mit einem Anschluss 10 zur Herstellung einer elektrischen Verbindung mit einem Hochspannungsgenerator 11 hat. Die Elektrodenanordnung 9 grenzt unmittelbar oder getrennt durch eine Zwischenschicht (z.B. Membran 8) oder einen Zwischenraum an das Wirkmedium 4 an. Die Elektrodenanordnung 9 ist somit zwischen Träger 3 und Wirkmedium 4 angeordnet. Sie kann zur flächigen Auflage auf eine zu behandelnde Oberfläche 1 flächig ausgebildet sein. Durch Anlegen einer Hochspannung z.B. in gepulster Form oder als Wechselspannung an die Elektrodenanordnung 9 wird Plasma erzeugt, mit dem das benachbarte Wirkmedium 4 bevorzugt direkt oder indirekt aktiviert wird. Dabei erfolgt die Entladung bevorzugt direkt in dem flüssigen oder gelförmigen Wirkmedium 4. Denkbar ist aber auch eine Entladung in einem Gasvolumen, das dann mit dem Wirkmedium 4 in Kontakt tritt, um so eine indirekte Plasmaaktivierung des Wirkmediums 4 zu bewirken.

Wiederum wird dieses aktivierte Wirkmedium 4 sodann in einem Applikationsschritt AP durch Auflegen der Wirkauflage 2 auf die zu behandelnde Oberfläche 1 auf die Oberfläche 1 appliziert.

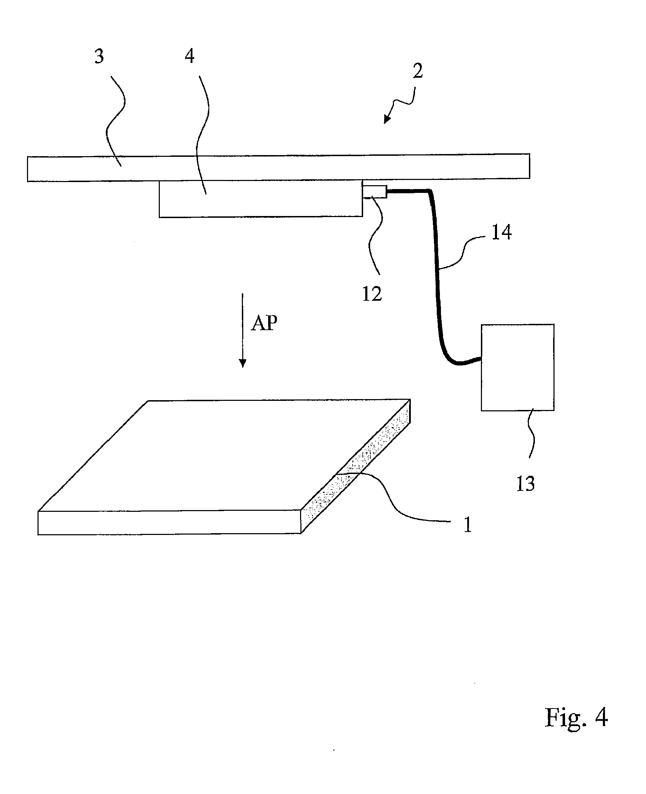

Figur 4 zeigt eine Skizze einer weiteren Ausführungsform zur Behandlung einer Oberfläche 1 mit einer Wirkauflage 2, die nunmehr zur Plasmaaktivierung einen mit dem Wirkmedium 4 auf dem Träger 3 kommunizierend verbundenen Anschluss 12 zum Anschluss einer Plasmaversorgungseinheit 13 hat. Die Plasmaversorgungseinheit 9 ist dabei zur Generierung einer aktiven Spezies z.B. durch Zündung von Plasma unter Niederdruck-, Normaldruck- oder Überdruckbedingungen ausgebildet. Dies kann z.B. in an sich bekannter Weise mittels Hochspannung erfolgen. Die so erzeugte plasmagenerierte aktive Spezies wird z.B. im flüssigen oder gasförmigen Zustand über eine Zuleitung 14 und den Anschluss 12 dem Wirkmedium 4 zugeführt, um dieses zu aktivieren und seine Wirkeigenschaften freizusetzen.

Wiederum wird dieses aktivierte Wirkmedium 4 sodann in einem Applikationsschritt AP durch Auflegen der Wirkauflage 2 auf die zu behandelnde Oberfläche 1 auf die Oberfläche 1 appliziert.

Denkbar ist bei den in Figur 2 bis 4 skizzierten Ausführungsformen auch, dass die Wirkauflage 1 bereits vor der Plasmaaktivierung des Wirkmediums 4 auf die zu behandelnde Oberfläche aufgelegt wird. Dabei kann die Plasmaaktivierung bedarfsweise auch während der Oberflächenbehandlung fortgesetzt werden.

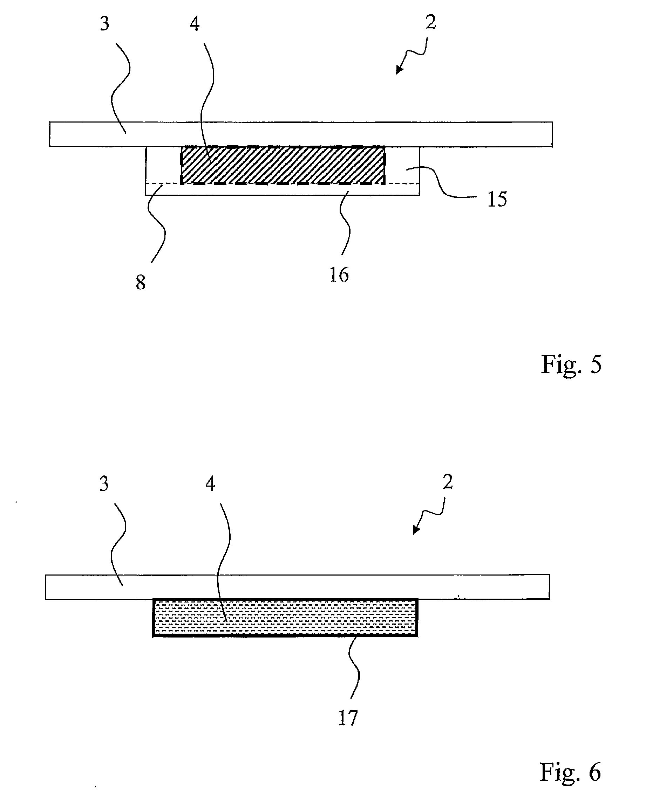

Figur 5 zeigt eine Schnittansicht einer Wirkauflage 2 mit einem Träger 3, der ein Auflagekissen 15 als Trägermaterial auf einer Auflagefläche trägt. Das Auflagekissen 15 umgibt ein plasmaaktivierbares Wirkmedium 4, das in dem von dem Auflagekissen 15 umschlossenen Innenraum aufgenommen ist. Das Wirkmedium 4 kann dabei z.B. im flüssigen oder gelförmigen Zustand sein, so dass das Auflagekissen 15 als Abdichtung dient und ein Austreten des Wirkmediums 4 verhindert. An der dem Träger 3 gegenüberliegenden Seite ist das Auflagekissen 15 und dass darin aufgenommene Wirkmedium 4 durch eine Membran 8 nach außen vollflächig abgeschlossen. An die Membran 8 kann sich optional eine Auflageschicht 16 anschließen, die zumindest für den nach der Plasmaaktivierung freigesetzten Wirkstoff des Wirkmediums 4 durchlässig ist.

Fig. 6 zeigt eine Schnittansicht einer Wirkauflage 2 mit einem Träger 3 und einer mit einer Trägerschicht 17 (Wirkauflageschicht), die mit plasmaaktivierbarem Wirkmedium getränkt ist. Diese Trägerschicht 17 kann dann aktiviert werden, indem diese einem Plasma ausgesetzt wird. Dies kann beispielsweise durch Beaufschlagen mit einem Plasmastrahl oder bevorzugt durch Einlegen der Wirkauflage 2 in einen mit Plasma gefüllten Behandlungsraum erfolgen.

## Patentansprüche

1. Wirkauflage (2) mit einem Wirkmedium (4), **dadurch gekennzeichnet, dass** das Wirkmedium (4) zur Aktivierung durch Plasma und zur Freisetzung eines Wirkstoffs nach der Plasmaaktivierung ausgebildet ist.

2. Wirkauflage (2) nach Anspruch 1, **dadurch gekennzeichnet, dass** die Wirkauflage (2) ein plasmaaktivierbares Gel, eine plasmaaktivierbare Flüssigkeit oder eine plasmaaktivierbare, fluidgetränkte Trägerschicht (17) hat.

3. Wirkauflage (2) nach Anspruch 1 oder 2, **dadurch gekennzeichnet, dass** die Wirkauflage (2) einen mit Gas befüllbaren Entladungsraum (7) hat, der zum Zünden eines Plasmas in dem Entladungsraum (7) eingerichtet ist.

4. Wirkauflage (2) nach einem der vorigen Ansprüche, **dadurch gekennzeichnet, dass** die Wirkauflage (2) eine mit dem Wirkmedium (4) gekoppelte Elektrodenanordnung (9) zur Plasmaaktivierung hat, wobei elektrische Kontakte (10) zum Anschluss der Elektrodenanordnung (9) an einen von der Wirkauflage (2) separaten Hochspannungsgenerator (11) vorgesehen sind.

5. Wirkauflage (2) nach einem der vorigen Ansprüche, **dadurch gekennzeichnet, dass** die Wirkauflage (2) einen mit dem Wirkmedium (4) kommunizierend verbundenen Anschluss (12) zum Anschluss einer von der Wirkauflage (2) separaten Plasmaversorgungseinheit (13) für die Zuleitung von plasmagenerierten aktiven Spezies von der Plasmaversorgungs-einheit (13) zum Wirkmedium (4) hat.

6. Wirkauflage (2) nach Anspruch 5, **dadurch gekennzeichnet, dass** die Plasmaversorgungseinheit (13) zur Zündung von Plasma unter Niederdruck-, Normaldruck oder Überdruckbedingungen ausgebildet ist.

7. Wirkauflage (2) nach einem der vorherigen Ansprüche, **dadurch gekennzeichnet, dass** die Wirkauflage (2) einen das Wirkmedium (4) tragenden Verbandsstoff hat und zur Auflage auf die zu behandelnde Oberfläche (1) vorgesehen ist.

8. Wirkauflage (2) nach einem der vorhergehenden Ansprüchen, **dadurch gekennzeichnet, dass** das Wirkmedium (4) nach einer Aktivierung durch Plasma mikrobiologisch desinfizierend ist.

9. Verfahren zur Behandlung einer Oberfläche (1), **gekennzeichnet durch**
- Aktivieren eines Wirkmediums (4) einer Wirkauflage (2) **durch** Beaufschlagung des Wirkmediums (4) mit einem Plasma;
und
- Applizieren des Wirkmediums (4) auf die zu behandelnde Oberfläche (1) **durch** Auflegen der Wirkauflage (2) auf die zu behandelnde Oberfläche (1).

10. Verfahren nach Anspruch 9, **gekennzeichnet durch** elektrisches Anschließen eines Hochspannungsgenerators (11) an eine Elektrodenanordnung (9) in der Wirkauflage (2) und Anlegen einer **durch** den separaten Hochspannungsgenerator (11) erzeugten Hochspannung an die Elektrodenanordnung (9) zur Erzeugung eines das Wirkmedium (4) aktivierenden Plasmas.

11. Verfahren nach Anspruch 9, **gekennzeichnet durch** Anschließen einer separaten Plasmaversorgungseinheit (13) an ein mit dem Wirkmedium (4) kommunizierend verbundenen Anschluss (12) der Wirkauflage (2) und Leiten von plasmagenerierten aktiven Spezies, das **durch** die Plasmaversorgungseinheit (13) zugeführt wird, zu dem Wirkmedium zur Aktivierung des Wirkmediums.

12. Verfahren nach Anspruch 11, **gekennzeichnet durch** Zünden von Plasma zur Generierung der plasmagenerierten aktiven Spezies unter Niederdruck-, Normaldruck oder Überdruckbedingungen.

13. Verfahren nach einem der Ansprüche 9 bis 12, **dadurch gekennzeichnet, dass** das Wirkmedium nach einer Aktivierung durch Plasma mikrobiologisch desinfizierend ist.
